# FASCICULE DE BREVET EUROPEEN

(11) **EP 3 030 225 B1**
(45) Date de publication et mention de la délivrance du brevet: **02.09.2020**
(21) Numéro de dépôt: 14780586.5
(22) Date de dépôt: 06.08.2014
(51) Int. Cl.: A61P 17/00, A61K 9/16, A61K 9/10, A61K 31/728, A61P 19/00, A61K 8/73, A61K 8/86, A61Q 19/08

(54) **COMPOSITIONS À LIBÉRATION CONTINUE À BASE D'ACIDE HYALURONIQUE, ET LEURS APPLICATIONS THÉRAPEUTIQUES**
ZUSAMMENSETZUNGEN AUF BASIS VON HYALURONSÄURE MIT KONTINUIERLICHER FREISETZUNG, UND DEREN THERAPEUTISCHE ANWENDUNGEN
CONTINUOUS RELEASE COMPOSITIONS MADE FROM HYALURONIC ACID, AND THERAPEUTIC APPLICATIONS OF SAME

(30) Priorité: 09.08.2013 WO PCT/EP2013/066763
(43) Date de publication de la demande: 15.06.2016
(73) Titulaire: Genbiotech, 06600 Antibes (FR)
(72) Inventeur: GUILLEMIN, Yannis, F-06510 GATTIERES (FR); VACHER, Dominique, F-06 250 Mougins (FR); PERRIER, Thomas, F-49124 Saint-Barthelemy d'Anjou (FR); GONNET, Maud, F-49100 Angers (FR); GOUZE, Jean-Noël, F-06220 Vallauris (FR)
(74) Mandataire: Cabinet Beau de Loménie
(86) Numéro de dépôt international: PCT/IB2014/063744
(87) Numéro de publication internationale: WO 2015/019304

(56) Documents cités:
- EP-A1- 0 911 025
- WO-A2-2008/147817
- US-A1- 2006 140 988
- US-B2- 7 456 275
- YADAV ET AL: "Development and characterization of hyaluronic acid-anchored PLGA nanoparticulate carriers of doxorubicin", NANOMEDICINE: NANOTECHNOLOGY, BIOLOGY AND MEDICINE, ELSEVIER, NL, vol. 3, no. 4, 18 décembre 2007 (2007-12-18), pages 246-257, XP022394224, ISSN: 1549-9634, DOI: 10.1016/J.NANO.2007.09.004

## Description

La présente invention concerne des particules de polymère constituées d'au moins un copolymère d'acide polylactique-co-glycolique-polyéthylène glycol-acide polylactique-co-glycolique (PLGA-PEG-PLGA), ou d'un mélange de polymère d'acide polylactique-co-glycolique (PLGA) et de copolymère d'acide polylactique-co-glycolique- polyéthylène glycol-acide polylactique-co-glycolique (PLGA-PEG-PLGA) dont le ratio en poids entre le polymère d'acide polylactique-co-glycolique (PLGA) et le copolymère d'acide polylactique-co-glycolique-polyéthylène glycol-acide polylactique-co-glycolique (PLGA- PEG-PLGA) varie de 40/60 à 60/40, associées à des molécules d'acide hyaluronique ou de sels d'acide hyaluronique, et leur procédé de préparation. La présente invention a également pour objet des compositions pharmaceutiques ou cosmétiques injectables comprenant de telles particules de polymère, le procédé de préparation de telles compositions, et leur utilisation à titre de médicament, notamment pour la prévention et/ou le traitement de maladies musculo-squelettiques, de maladies et des états traumatiques de la peau, des affections bucco-dentaires, de la sécherese de la muqueuse vaginale et des infections urinaires ou cystites, de la sécheresse de la membrane oculaire et des infections oculaires, et de l'obésité, ainsi que leur utilisation pour lutter contre le vieillissement de la peau et/ou pour réparer le tissu dermique (mésothérapie). Plus particulièrement, les compositions de l'invention peuvent être destinées à complémenter des liquides articulaires, et en particulier du liquide synovial, en rétablissant les conditions physiologiques et rhéologiques de l'articulation pathologique, par exemple arthrosique, ou encore pour les soins et la réparation de l'épiderme par remodelage, hydratation et protection de la peau. Enfin, l'invention vise un kit constitué de deux contenants, dont l'un au moins comprend des particules de polymère selon l'invention, et vise également une seringue prête à l'emploi comprenant une composition selon l'invention.

L'arthrose se caractérise par une invalidité et perte de motricité. Les articulations concernées sont en premier lieu le genou, mais aussi l'épaule, les vertèbres cervicales et la hanche. Des injections locales intra-articulaires d'acide hyaluronique peuvent être utilisées comme thérapie par viscosupplémentation contre ces phénomènes arthrosiques douloureux (type gonarthrose sans épanchement).

Les injections d'acide hyaluronique occupent également une place importante en cosmétique pour traiter le vieillissement physiologique de la peau ou des traumatismes de la peau, en assurant la réparation des tissus.

L'intérêt principal des compositions injectables à base d'acide hyaluronique réside dans le maintien de l'ensemble des propriétés de l'acide hyaluronique, à savoir :
- au niveau cellulaire et biochimique, des propriétés anti-apoptotiques (Ponta H. et al., Nature Reviews, Molecular Cell Biology, 2003, Vol. 4, 33-45 ; Ando A. et al., Tohoku J. Exp. Med., 2008, 215, 321-331 ; Zhou PH et al., Journal of Orthopaedic Research, 2008, 1643-1648), anti-inflammatoires (Vignon *et al.* 1997, 47, S11-S15 ; Ando A. *et al.* 2008 ; Zhou PH et al., Journal of Orthopaedic Research, 2008, 1643-1648), antioxydantes (Mendoza G. et al., Mini-Reviews in Médicinal Chemistry, 2009, 9, 1479-1488), sur l'amélioration de la synthèse d'acide hyaluronique par les synoviocytes (Nagaoka I. et al., Advances in Food and Nutrition Research, 2012, Vol. 65, Chap. 22), anti-fibrotiques (Li J. et al., Arthritis Research & Therapy, 2012, 14:R151), hydratantes (Masson, F., Annales de dermatologie, 2010, 137, Supplément 1, S23-S25 ; Hargittai I. et al. Struct. Chem., 2008, 19, 697-717),
- au niveau biomécanique : viscosupplémentation (Bellamy N. et al., Viscosupplementation for the treatment of osteoarthritis of the knee (Review), 2006, The Cochrane Collaboration®, John Wiley & Sons).

Dans les pathologies de l'articulation, les solutions injectables d'acide hyaluronique sont des outils pharmaceutiques efficaces et bien tolérés. Ces solutions évitent une dégradation rapide de l'acide hyaluronique, et prolongent son efficacité au point d'injection. Il en résulte une nette amélioration de la fonctionnalité des articulations pendant une période de temps prolongée. De plus, l'acide hyaluronique est parfaitement bien toléré dans l'articulation et ne présente pas d'immunogénicité.

A l'heure actuelle, les injections d'acide hyaluronique requièrent un rythme d'injection d'une à cinq injections, par articulation ou sous-cutané, à une semaine d'intervalle. Elles présentent un effet dans le cas de l'articulation qui ne dépasse pas 6 à 8 mois d'efficacité. En outre, même si l'injection d'acide hyaluronique non réticulé produit un effet rapide de désensibilisation des articulations, on constate qu'en moyenne au bout de 24h il ne reste plus aucune trace d'acide hyaluronique au site d'injection.

Le Brevet US 7,456,275 B2 décrit une préparation pharmaceutique de faible viscosité composée d'acide hyaluronique lié par modification chimique à un polymère bloc, cette préparation présentant un effet antalgique prolongé. La méthode mise en œuvre pour fabriquer une telle préparation présente l'inconvénient de modifier la structure chimique du principe actif acide hyaluronique, par création de liaisons covalentes fortes entre l'acide hyaluronique et le polymère. En outre, l'acide hyaluronique ainsi modifié chimiquement n'est pas soluble en milieu aqueux, mais uniquement dans des milieux organiques comme le diméthylsulfoxyde. Ainsi, il est nécessaire de réaliser une émulsion dans une huile minérale avant l'injection, l'ajout d'un tel solvant dénaturant en partie le principe actif.

La Demande WO 2006/071694 décrit une émulsion d'acide hyaluronique associée à un polymère biodégradable utilisée comme produit de viscosupplémentation pour améliorer les douleurs articulaires. L'acide hyaluronique est présent dans des gouttelettes dispersées dans une phase polymérique organique, l'utilisation de solvants organiques et de tensioactifs pouvant s'avérer néfaste. La méthode de préparation d'une telle émulsion montre également des problèmes de reproductibilité dans la mesure où la taille des gouttelettes formées ne peut être maîtrisée. Enfin, un autre inconvénient de cette méthode réside dans la nécessité d'un stockage à froid pendant plusieurs jours, une fois l'émulsion préparée.

Les Inventeurs ont maintenant mis au point un procédé de préparation de particules de polymère associées à des molécules d'acide hyaluronique ou de sels d'acide hyaluronique, qui permet de surmonter les inconvénients des compositions décrites dans l'art antérieur. Les compositions comprenant les particules de polymère de l'invention présente un profil de libération de l'acide hyaluronique plus lent que les compositions de l'art antérieur (diffusion de l'acide hyaluronique prolongée dans le temps), la durée de disponibilité de l'acide hyaluronique étant par conséquent plus longue. La composition de l'invention a un effet continu dans le temps, qui réduit la fréquence des injections articulaires, les compositions de l'invention pouvant être injectées une seule fois en un an. L'acide hyaluronique associé aux particules de polymère se diffuse alors à petites doses, pendant plusieurs mois, comme s'il s'agissait d'une « perfusion » continue d'acide hyaluronique, par exemple dans l'articulation, réduisant de manière importante le nombre d'injections. La composition de l'invention présente en outre l'avantage d'être une composition lyophilisée stérile stable qui peut être utilisée en une seule seringue, contrairement à la composition de la Demande WO 2006/071694.

Ainsi, la présente invention a pour premier objet des particules de polymère constituées d'au moins un copolymère d'acide polylactique-co-glycolique-polyéthylène glycol-acide polylactique-co-glycolique (PLGA-PEG-PLGA), ou d'un mélange de polymère d'acide polylactique-co-glycolique (PLGA) et de copolymère d'acide polylactique-co-glycolique- polyéthylène glycol-acide polylactique-co-glycolique (PLGA-PEG-PLGA) dont le ratio en poids entre le polymère d'acide polylactique-co-glycolique (PLGA) et le copolymère d'acide polylactique-co-glycolique-polyéthylène glycol-acide polylactique-co-glycolique (PLGA- PEG-PLGA) varie de 40/60 à 60/40, associées à des molécules d'acide hyaluronique ou de sels d'acide hyaluronique.

La structure du polymère PLGA est représentée ci-après :

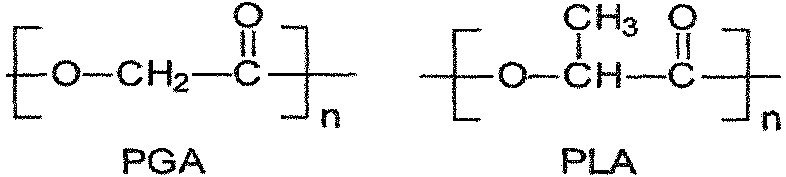

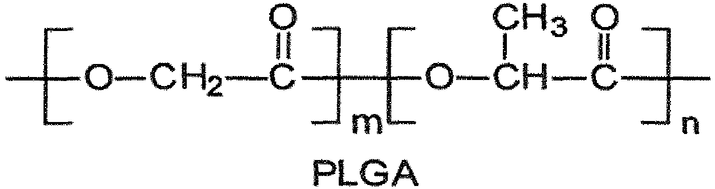

L'acide hyaluronique de l'invention peut se présenter soit sous forme réticulé, soit sous forme non réticulée. L'acide hyaluronique réticulé consiste en deux ou plusieurs molécules d'acide hyaluronique unies entre elles par des liaisons covalentes, formant ainsi une masse gélifiée d'acide hyaluronique cohésive et stable (De Boulle K. et al., Dermatologie Surgery, 2013, 39 : 1758-1766 ; Schanté C. E. et al., Carbohydrate Polymers, 85, 2011, 469-489). De préférence l'acide hyaluronique de l'invention se présente sous forme non réticulée.

Lorsque les molécules d'acide hyaluronique se présentent sous forme de sels, il s'agit de préférence de sels de sodium.

Les molécules d'acide hyaluronique ou de sels d'acide hyaluronique sont de préférence associées au polymère d'acide polylactique-co-glycolique-polyéthylène et/ou au copolymère d'acide polylactique-co-glycolique-polyéthylène glycol-acide polylactique-co-glycolique, par des liaisons de faible énergie, et de préférence par des liaisons hydrogène et/ou des liaisons de Van der Waals. En effet, le procédé de préparation des particules de polymère de l'invention permet d'associer les molécules d'acide hyaluronique au copolymère d'acide polylactique-co-glycolique-polyéthylène glycol-acide polylactique-co-glycolique (PLGA-PEG-PLGA), ou d'un mélange de polymère d'acide polylactique-co-glycolique (PLGA) et de copolymère d'acide polylactique-co-glycolique- polyéthylène glycol-acide polylactique-co-glycolique (PLGA-PEG-PLGA) dont le ratio en poids entre le polymère d'acide polylactique-co-glycolique (PLGA) et le copolymère d'acide polylactique-co-glycolique-polyéthylène glycol-acide polylactique-co-glycolique (PLGA- PEG-PLGA) varie de 40/60 à 60/40, sans modification chimique de la structure de l'acide hyaluronique, c'est-à-dire sans formation de liaisons covalentes entre l'acide hyaluronique et le polymère. Ainsi, le procédé de l'invention évite l'emploi d'agents activants et d'agents de couplage, telle que l'hydrazine, normalement nécessaire pour modifier la structure de l'acide hyaluronique et créer des liaisons covalentes (Schanté C. E. et al., Carbohydrate Polymers, 85, 2011, 469-489).

Selon un mode de réalisation préférée, les particules de polymère de l'invention sont constituées d'un mélange de polymère d'acide polylactique-co-glycolique (PLGA) et de copolymère d'acide polylactique-co-glycolique-polyéthylène glycol-acide polylactique-co-glycolique (PLGA-PEG-PLGA). De manière avantageuse, le ratio en poids entre le polymère d'acide polylactique-co-glycolique (PLGA) et le copolymère d'acide polylactique-co-glycolique-polyéthylène glycol-acide polylactique-co-glycolique (PLGA-PEG-PLGA) varie de 40/60 à 60/40, et de préférence est de 50/50.

Selon un mode de réalisation encore plus préféré, les particules de polymère de l'invention sont constituées uniquement de copolymère d'acide polylactique-co-glycolique-polyéthylène glycol-acide polylactique-co-glycolique (PLGA-PEG-PLGA) (100% PLGA-PEG-PLGA).

Le polyéthylène glycol (PEG) a de préférence un poids moléculaire élevé allant de 4 000 à 10 000 g.mol⁻¹. Le PLGA a de préférence un poids moléculaire allant de 54 000 à 69 000 g.mol⁻¹. Le copolymère d'acide polylactique-co-glycolique-polyéthylène glycol-acide polylactique-co-glycolique (PLGA-PEG-PLGA) a de préférence un poids moléculaire allant de 50 000 à 70 000 g.mol⁻¹. Ces polymères sont disponibles commercialement par exemple sous les références LSB 5050 DLG/PEG 6000 et Resomer RG 505 de la Société Evonik.

Les particules de polymère de l'invention ont de préférence une taille allant de 10 à 130 µm, et de manière plus préférée allant de 20 à 85 µm. La taille des particules peut être déterminée par polydispersité selon la granulométrie à l'aide d'un compteur Coulter Multisizer® 3 (Beckman Coulter).

Les molécules d'acide hyaluronique ou de sels d'acide hyaluronique associées aux particules de polymère ont quant à elles de préférence un poids moléculaire pouvant varier de 0,8.10⁶ à 1,2.10⁶ g.mol⁻¹, déterminées par exemple par chromatographie d'exclusion stérique (SEC) ou chromatographie liquide haute performance (HPLC), les molécules d'acide hyaluronique ou de sels d'acide hyaluronique présentes dans le commerce ayant un poids moléculaire variant de 0,5.10⁶ à 6,0.10⁶ g.mol⁻¹.

De manière avantageuse, le taux en poids d'acide hyaluronique ou de sels d'acide hyaluronique associé aux particules de polymère varie de 1 à 50 µg.mg⁻¹, de préférence de 5 à 30 µg.mg⁻¹, et encore plus préférentiellement de 10 à 30 µg.mg⁻¹.

Un autre objet de l'invention concerne un procédé de préparation de particules de polymère telles que définies selon l'invention, ledit procédé comprenant les étapes suivantes :
(i) sous agitation, mise en émulsion d'une solution aqueuse d'acide hyaluronique ou de sels d'acide hyaluronique dans une solution organique comprenant au moins un copolymère d'acide polylactique-co-glycolique-polyéthylène glycol-acide polylactique-co-glycolique (PLGA-PEG-PLGA), ou d'un mélange de polymère d'acide polylactique-co-glycolique (PLGA) et de copolymère d'acide polylactique-co-glycolique- polyéthylène glycol-acide polylactique-co-glycolique (PLGA-PEG-PLGA) dont le ratio en poids entre le polymère d'acide polylactique-co-glycolique (PLGA) et le copolymère d'acide polylactique-co-glycolique-polyéthylène glycol-acide polylactique-co-glycolique (PLGA- PEG-PLGA) varie de 40/60 à 60/40,
(ii) sous agitation, mélange de l'émulsion obtenue à l'issue de l'étape (i) avec une solution aqueuse d'alcool polyvinylique (PVA),
(iii) sous agitation, ajout d'un solvant aqueux dans l'émulsion obtenue à l'issue de l'étape (ii), ledit solvant aqueux mis en œuvre étant de préférence de l'eau purifiée,
(iv) filtration de l'émulsion obtenue à l'issue de l'étape (iii), afin d'éliminer la phase aqueuse,
(v) lyophilisation des particules de polymère obtenues à l'issu de l'étape (iv), cette étape permettant l'élimination des trace d'eau éventuellement présentes dans les particules de polymère obtenues à l'issue de l'étape (iv),
(vi) éventuellement, stérilisation des particules de polymère lyophilisées obtenues à l'issue de l'étape (v).

Lors de l'étape (i), la solution aqueuse d'acide hyaluronique ou de sels d'acide hyaluronique peut être à base d'eau purifiée, et éventuellement d'alcool polyvinylique (PVA), de préférence il s'agit d'une solution aqueuse d'alcool polyvinylique (PVA) ayant une concentration en poids en alcool polyvinylique (PVA) de 1 à 10%, de préférence de 2 à 5%, et encore plus préférentiellement de 4%. Le polymère d'acide polylactique-co-glycolique (PLGA) et/ou le copolymère d'acide polylactique-co-glycolique-polyéthylène glycol-acide polylactique-co-glycolique (PLGA-PEG-PLGA) est de préférence en solution dans un solvant organique choisi parmi le dichlorométhane, l'acétone, ou leur mélange. L'étape (i) est de préférence réalisée sous agitation à l'aide d'un agitateur de type Ultra-Thurrax® ou d'une source d'ultrasons. L'étape (i) peut être conduite à température ambiante, puis à froid dans un bain de glace.

La solution aqueuse d'alcool polyvinylique (PVA) mise en œuvre lors de l'étape (ii) a de préférence une concentration en poids en alcool polyvinylique (PVA) de 1 à 10%, avantageusement de 2 à 5%, et encore plus avantageusement de 4%. Ledit alcool polyvinylique (PVA) a de préférence un poids moléculaire allant de 30 000 à 50 000 g.mol⁻¹. Le ratio en volume entre la solution aqueuse d'alcool polyvinylique (PVA) et l'émulsion obtenue à l'issu de l'étape (i) varie avantageusement entre 20/1 et 40/1.

Lors de l'étape (ii), l'émulsion obtenue à l'issue de l'étape (i) peut être injectée à l'aide d'une seringue dans un réacteur, de préférence dans un homogénéisateur de type Silverson ou un réacteur à symétrie cylindrique, comprenant la solution aqueuse d'alcool polyvinylique (PVA). L'agitation peut être maintenue pendant 1 à 10 minutes, avant d'ajouter un solvant aqueux à l'émulsion (étape (iii)). L'agitation peut ensuite à nouveau être maintenue pendant 5 à 15 minutes, afin que le solvant contenu dans les gouttelettes formées diffuse dans la solution aqueuse contenant l'alcool polyvinylique (PVA), et conduise à l'association de l'acide hyaluronique aux particules de polymère. Eventuellement, le solvant contenu dans l'émulsion obtenue à l'issue de l'étape (iii) peut ensuite être évaporé, avant d'être filtré selon l'étape (iv).

De préférence, l'étape (iv) de filtration est réalisée sur un filtre hydrophile tels que les filtres HVLP 0,45 µm ou SSWP 3 µm.

Après l'étape (iv), les particules de polymère obtenues peuvent être lavées avec de l'eau purifiée.

Après cette éventuelle étape de lavage, les particules de polymère obtenues peuvent être conditionnées dans un flacon ou une seringue, avant d'être lyophilisées.

Selon un mode de réalisation préféré, après cette étape de conditionnement, les particules de polymère selon l'invention peuvent être mises en contact avec une solution de reprise comprenant un excipient pharmaceutiquement ou cosmétiquement acceptable et/ou un autre principe actif (autre que l'acide hyaluronique), cette dernière étant ajoutée aux particules de polymère obtenues à l'issu de l'étape (iv) et après l'éventuelle étape de lavage, avant lyophilisation. La solution de reprise de l'invention peut être de l'eau purifiée, une solution aqueuse tamponnée d'acide hyaluronique (réticulé ou non réticulé) ou de sels d'acide hyaluronique, ou une solution aqueuse d'un autre sel tel que le chlorure de sodium (NaCl), ladite solution de reprise pouvant également contenir, en fonction de l'application finale visée, un ou plusieurs solvants choisis parmi l'éthanol, le propylèneglycol, le polyéthylèneglycol, le squalène, les huiles végétales telles que les huiles à base de mono-, di-, tri-glycérides, les huiles minérales, l'huile de foie de morue. Cette solution de reprise est de préférence une solution isotonique, dont la nature dépend de l'application finale visée, Par exemple, il peut s'agir d'une solution aqueuse d'acide hyaluronique non réticulé ou de sels d'acide hyaluronique lorsque l'application finale vise à prévenir et/ou traiter l'arthrose ; une solution aqueuse d'acide hyaluronique réticulé pour le comblement des rides ; une solution aqueuse de chlorure de sodium (NaCl) pour la mésothérapie. De manière avantageuse, la solution de reprise mise en œuvre après l'étape (iv), et éventuellement après l'étape de conditionnement, est de l'eau purifiée comprenant éventuellement du sorbitol, du mannitol, ou du tréhalose, et de préférence du sorbitol, en tant qu'excipient pharmaceutiquement acceptable, et un agent anesthésique en tant que principe actif.

L'expression «excipient pharmaceutiquement ou cosmétiquement acceptable» désigne des diluants, des adjuvants ou des véhicules, tels que des conservateurs, des charges, des agents de désintégration, des agents mouillants, des agents émulsifiants, des agents de mise en suspension, des solvants, des dispersants, des lubrifiants, des revêtements, des agents antibactériens et antifongiques, des agents isotoniques, des retardateurs d'absorption et leurs analogues. L'excipient pharmaceutiquement ou cosmétiquement acceptable de l'invention est de préférence choisi parmi le sorbitol, le mannitol, le tréhalose, le lactose, et de préférence le sorbitol. D'autres excipients pharmaceutiquement ou cosmétiquement acceptables peuvent être envisagés en fonction de l'application finale visée, par exemple pour modifier la couleur ou le goût de la composition de l'invention si celle-ci à des visées bucco-dentaires.

Le principe actif supplémentaire peut être un agent anesthésique tel que la lidocaïne.

Certains excipients pharmaceutiquement acceptables tels que le sorbitol peuvent à la fois jouer le rôle d'excipient pharmaceutiquement acceptable et de principe actif supplémentaire, ledit sorbitol présentant en plus de son rôle d'excipient, des propriétés anti-inflammatoires (capteur de radicaux libres) (Mongkhon J-M. et al., Inflamm. Res., Springer, 2014).

L'étape (v) de lyophilisation est avantageusement réalisée selon le cycle suivant :
- diminution de la température à une température de -38°C pendant une durée allant de 30 minutes à lh30,
- augmentation de la température à une température allant de -38°C à +15°C pendant une durée allant de 1h30 à 2h30,
- séchage à une température de +15°C pendant une durée allant de 4 à 6h,
- augmentation de la température à une température allant de +15 à +25°C pendant une durée allant de 15 à 45 minutes, puis
- séchage à une température de +25°C pendant une durée de 8 à 12h.

Les particules de polymère lyophilisées peuvent ensuite être stérilisées par stérilisation aux rayons gamma lors d'une étape (vi). Ce type de stérilisation permet d'éviter toute détérioration de la structure, et toute altération des propriétés des particules de polymère obtenues.

Une composition, de préférence une composition pharmaceutique ou cosmétique, comprenant des particules de polymère telles que définies selon l'invention constitue un autre objet de l'invention. Dans une telle composition, les particules de polymère sont en suspension dans une solution de reprise telle que définie précédemment, ladite solution de reprise pouvant être identique ou différente de celle utilisée après l'étape de lavage réalisée après l'étape (iv). De manière avantageuse, la solution de reprise présente dans la composition de l'invention est de l'eau purifiée, une solution aqueuse tamponnée d'acide hyaluronique (réticulé ou non réticulé) ou de sels d'acide hyaluronique, ou une solution aqueuse d'un autre sel tel que le chlorure de sodium (NaCl). De manière encore plus préférée, la solution de reprise présente dans la composition de l'invention est une solution aqueuse tamponnée d'acide hyaluronique (réticulé ou non réticulé) ou de sels d'acide hyaluronique, de préférence a une concentration en poids d'acide hyaluronique ou de sels d'acide hyaluronique allant de 0,5 à 10%, et de préférence de 0,5 à 4%.

Selon un mode de réalisation avantageux, la composition de l'invention peut également comprendre un excipient pharmaceutiquement ou cosmétiquement acceptable tel que défini précédemment. Selon un autre mode de réalisation avantageux, la composition de l'invention peut également comprendre un autre principe actif. Ledit principe actif supplémentaire est tel que défini précédemment.

L'invention concerne également un procédé de préparation d'une composition telle que définie selon l'invention, ledit procédé comprenant les étapes (i) à (vi) telles que définies précédemment, et une étape (vii) de mise en suspension des particules de polymère lyophilisées obtenues à l'issue de l'étape (v) ou (vi) dans une solution de reprise telle que définie précédemment, ladite solution de reprise pouvant également comprendre un excipient pharmaceutiquement ou cosmétiquement acceptable et/ou un autre principe actif. De manière avantageuse, la solution de reprise mise en oeuvre lors de l'étape (vii) est de l'eau purifiée, une solution aqueuse tamponnée d'acide hyaluronique (réticulé ou non réticulé) ou de sels d'acide hyaluronique, ou une solution aqueuse d'un autre sel tel que le chlorure de sodium (NaCl). De manière encore plus préférée, la solution de reprise mise en œuvre lors de l'étape (vii) est une solution aqueuse tamponnée d'acide hyaluronique (réticulé ou non réticulé) ou de sels d'acide hyaluronique, de préférence a une concentration en poids d'acide hyaluronique ou de sels d'acide hyaluronique allant de 0,5 à 10%, et de préférence de 0,5 à 4%.

La présente invention a également pour objet une composition selon l'invention pour son utilisation à titre de médicament, et de préférence pour la prévention et/ou le traitement des maladies musculo-squelettiques, des maladies et des états traumatiques de la peau, tels que les brûlures, des affections bucco-dentaires, de la sécheresse de la muqueuse vaginale et des infections urinaires, des infections oculaires, et de l'obésité lors de la pose d'anneaux gastriques, et également pour la prévention et/ou le traitement des maladies rhumatologiques chez l'animal, et plus particulièrement des chevaux de course.

Plus particulièrement, l'invention a pour objet la prévention et/ou le traitement :
- de maladies musculo-squelettiques telles que l'arthrose, et plus particulièrement de la gonarthrose ou de l'arthrose sans épanchement,
- d'affections bucco-dentaires choisies parmi la sécheresse des muqueuses buccales (WO 2012/093753 A1), les inflammations et les états traumatiques de la muqueuse buccale,
- de la sécheresse de la muqueuse vaginale et des infections urinaires ou cystites (WO 1996/025168 A1 ; EP 0813417 A1 ; S. Van Agt et al., Progrès en urologie, Vol. 21, Issue 3, 2011, pp. 218-225), et
- de la sécheresse de la membrane oculaire et des infections oculaires.

La composition de l'invention peut également être utilisée pour lutter contre le vieillissement de la peau et/ou pour réparer le tissu dermique (mésothérapie).

Enfin, l'invention concerne un kit comprenant :
(a) un premier contenant comprenant des particules de polymère telle que définies selon l'invention, et
(b) un deuxième contenant comprenant une solution de reprise telle que définie précédemment, ladite solution de reprise pouvant également comprendre un excipient pharmaceutiquement ou cosmétiquement acceptable et/ou un autre principe actif tels que définis précédemment.

Selon un mode de réalisation avantageux du kit de l'invention, plus particulièrement destiné à la prévention et/ou au traitement de maladies musculo-squelettiques, le premier contenant est un flacon ou une seringue, et le deuxième contenant est une seringue, lesdits contenants étant liés entre eux par un connecteur adapté.

Ainsi, les compositions de l'invention peuvent être présentées soit en un seul module d'injection, soit en deux parties (kit), et dans ce dernier cas être mélangées extemporanément, avant injection *in situ,* soit par voie intra-articulaire pour le traitement des articulations, soit par voie sous-cutanée pour la réparation de la peau.

Un dernier objet de l'invention vise une seringue prête à l'emploi comprenant une composition telle que définie selon l'invention. Une telle seringue est particulièrement adaptée pour une utilisation cosmétique, par exemple pour les soins et la réparation de l'épiderme par remodelage ou hydratation.

Outre les dispositions qui précèdent, l'invention comprend encore d'autres dispositions qui ressortiront du complément de description qui suit, qui se rapporte à des exemples de préparations de particules de polymère et de compositions selon l'invention, et à l'évaluation *in vivo* de la tolérance de ces compositions lors d'injections dans l'espace intra-articulaire chez le lapin, ainsi qu'aux dessins annexés dans lesquels :
- les **Figures 1a** et **1b** sont respectivement des images de particules de polymère dans lesquels sont liées des molécules d'acide hyaluronique selon l'invention préparées selon l'Exemple 1 et l'Exemple 2 (images de microscopie électronique à balayage),

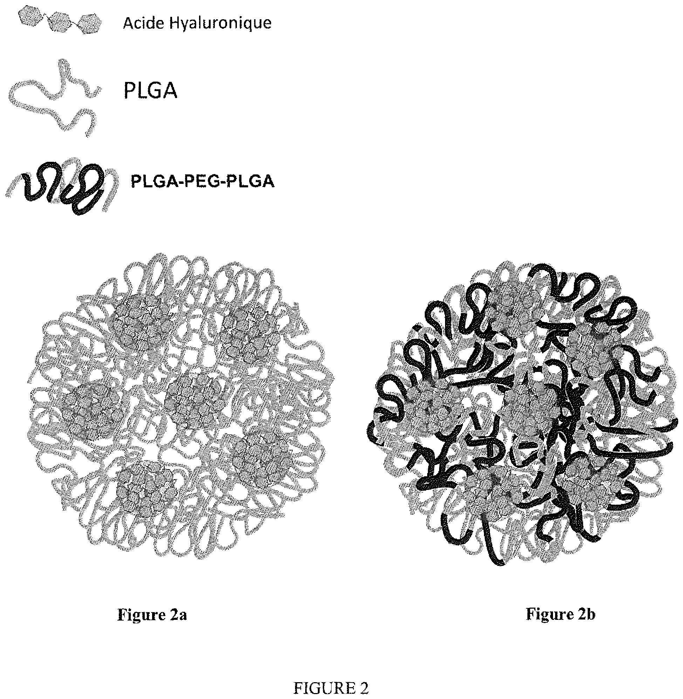

- les **Figures 2a** et **2b** représentent respectivement des particules de polymère de PLGA selon l'invention associées à des molécules d'acide hyaluronique **(****Figure 2a****),** et des particules de polymère de PLGA-PEG-PLGA selon l'invention associées à des molécules d'acide hyaluronique **(****Figure 2b****),**
- la **Figure 3** représente le profil de libération des particules de polymère associées à des molécules d'acide hyaluronique, préparé selon l'Exemple 1, et
- la **Figure 4** représente le profil de libération des particules de polymère associées à des molécules d'acide hyaluronique, préparées selon l'Exemple 2.

### EXEMPLES :

Matières premières utilisées :

**Tableau 1 :**

| **Réactifs** | **Références commerciales** | **Fournisseurs** |
|---|---|---|
| Acide hyaluronique | AH HMW | Altergon |
| Alcool polyvinylique (PVA) | Gohsenol EG-05PW | Nippon Gohseï |
| Tribloc PLGA-PEG-PLGA | LSB 5050 DLG PEG 6000 | Evonik (Birmingham Labs) |
| PLGA | Resomer RG 505 | Evonik |
| Eau ultra-pure | - | MilliPore |
| Acétone | 24201 | Sigma Aldrich |
| Dichlorométhane | 24233 | Sigma Aldrich |
| Sorbitol | S1876 | Sigma Aldrich |

### Exemple 1 :

### Préparation des particules de polymère selon l'invention :

### 1^{ère} étape : émulsification « primaire »

Une solution aqueuse d'acide hyaluronique est préparée en dissolvant 50 mg d'acide hyaluronique dans 5 mL d'une solution d'alcool polyvinylique (PVA) à 4% en poids. Une solution organique de polymère est également préparée en dissolvant 900 mg d'un polymère tribloc PLGA-PEG-PLGA dans 12 mL d'un mélange dichlorométhane/acétone (3/1 v/v). Ces deux solutions sont émulsionnées pendant deux minutes à température ambiante avec un agitateur de type Ultra-Turrax® IKA T25 Basic à une vitesse de 16 000 tours/minute, et simultanément avec une agitation magnétique à l'aide d'un barreau aimanté à une vitesse de 500 tours/minute. Un second cycle d'agitation est réalisé à l'identique dans un bain de glace.

### 2^{ème} étape : émulsification « secondaire »

L'émulsion stable obtenue à l'issue de la 1^{ère} étape est introduite dans une seringue en verre, puis injectée dans un réacteur de formulation contenant 450 mL d'alcool polyvinylique (PVA) à 4% en poids, sous agitation magnétique à une vitesse de 750 tours/minutes.

### 3^{ème} étape : ajout d'un solvant aqueux

L'agitation est maintenue pendant deux minutes, puis 500 mL d'eau ultra-pure (résistivité supérieure à 18 MΩ.cm⁻¹) sont ajoutés dans le réacteur. L'agitation est ensuite maintenue pendant dix minutes supplémentaires, à une vitesse de 750 tours/minute. Le solvant contenu dans les gouttelettes préalablement formées se diffuse alors dans la phase aqueuse contenant l'alcool polyvinylique (PVA), et il se forme alors des particules de polymère associées à des molécules d'acide hyaluronique.

### 4^{ème} étape : purification et extraction du solvant

Le solvant présent dans l'émulsion obtenue à l'issue de la 3^{ème} étape est ensuite évaporé par agitation magnétique à une vitesse de 500 tours/minute, pendant 4h, à 20°C, sous hotte.

Les particules de polymère chargées d'acide hyaluronique sont ensuite filtrées sur un filtre hydrophile SSWP 3 µm afin d'éliminer la phase aqueuse. Les particules obtenues sont ensuite lavées avec de l'eau ultra-pure, à raison de 3 L pour 900 mg de particules.

Les particules de polymère sont ensuite conditionnées dans des flacons en verre ambré de hauteur 50 mm et de diamètre 24 mm (150 mg de particules par flacon). Les particules sont ensuite recouvertes avec 1 mL d'eau ultra-pure pour 150 µg de particules de polymère.

### 5^{ème} étape : lyophilisation et stérilisation

Les particules de polymère contenues dans les flacons sont ensuite lyophilisées pour obtenir un lyophilisat de particules sèches. La lyophilisation est réalisée selon le cycle suivant : diminution de la température à une température de -38°C pendant 1h, puis augmentation de la température de -38°C à +15°C pendant 2h, suivi d'un séchage à +15°C pendant 5h, puis augmentation de la température de +15°C à +25°C pendant 30 min, suivi d'un séchage à +25°C, pendant 10h.

Puis les particules chargées d'acide hyaluronique lyophilisées sont ensuite stérilisées par irradiation au rayonnement gamma à 15 kGy.

### 6^{ème} étape :

150 mg de particules de polymère sont mises en solution dans 1 mL d'une solution aqueuse stérile comprenant 2 mL de NaCl dans lesquels sont dissous 16 mg d'acide hyaluronique. La mise en solution est réalisée avec un système de seringue Luer-Lock®.

### Analyse des particules de polymère de l'invention selon l'Exemple 1 :

Les analyses ont été réalisées sur 10 lots de particules de polymère selon l'invention. Les résultats obtenus sont les suivants :
- taille des particules déterminée par polydispersité selon la granulométrie à l'aide d'un compteur Coulter Multisizer® 3 (Beckman Coulter) : 66,34 µm ± 3,6 µm (avant irradiation), et 65,34 µm ± 7,09 µm (après irradiation),
- un taux de charge en acide hyaluronique de 13 µg d'acide hyaluronique par mg de particules de polymère lyophilisées, et
- un rendement d'association des molécules d'acide hyaluronique aux particules de polymère d'environ 40%.

Les résultats obtenus sont récapitulés dans le **Tableau 2** (taille des particules chargées en acide hyaluronique, rendement d'association des molécules d'acide hyaluronique aux particules de polymère, taux de charge des particules en acide hyaluronique).

**Tableau 2 :**

| | | Taille Moyenne (µm) | | Rendement d'association des molécules d'acide hyaluronique aux particules de polymère (%) | | Taux de charge (µg/mg) | |
|---|---|---|---|---|---|---|---|
| | | Avant irradiation | Après irradiation | Avant irradiation | Après irradiation | Avant irradiation | Après irradiation |
| Lots | 1 | 65,87 | 61,74 | 50 | 51 | 16,20 | 16,53 |
| | 2 | 72,13 | 73,45 | 57 | 55 | 18,47 | 17,82 |
| | 3 | 62,54 | 65,89 | 57 | 52 | 18,47 | 16,85 |
| | 4 | 64,58 | 70,87 | 40 | 38 | 12,96 | 12,31 |
| | 5 | 61,32 | 58,44 | 29 | 27 | 9,40 | 8,75 |
| | 6 | 67,12 | 69,78 | 29 | 28 | 9,40 | 9,07 |
| | 7 | 71,45 | 76,44 | 40 | 38 | 12,96 | 12,31 |
| | 8 | 69,63 | 62,77 | 42 | 40 | 13,61 | 12,96 |
| | 9 | 63,82 | 55,23 | 37 | 36 | 11,99 | 11,67 |
| | 10 | 64,97 | 58,75 | 35 | 38 | 11,34 | 12,31 |
| **Moyenne** | | 66,34 | 65,34 | 41,60 | 40,30 | 13,48 | 13,06 |
| **Ecart-type** | | 6,69 | 7,09 | 10,18 | 9,60 | 3,30 | 3,11 |

Des images de particules de polymère associées à des molécules d'acide hyaluronique selon l'invention préparées selon l'Exemple 1 sont représentées à la **Figure la.**

### Résultats :

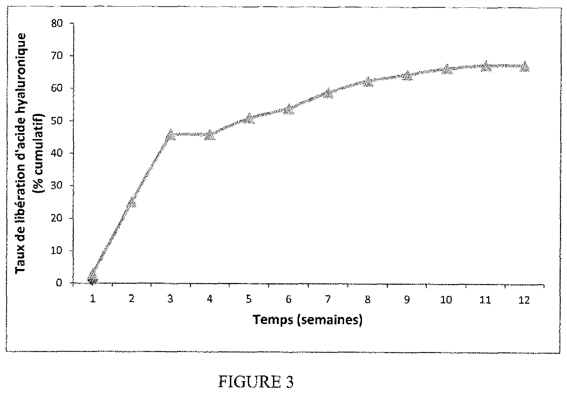

Le profil de libération des particules de polymère liées à des molécules d'acide hyaluronique préparées selon l'Exemple 1 est représenté à la **Figure 3****.** Les mesures ont été réalisées en milieu bio-mimétique, en reconstituant un milieu proche du liquide synovial (milieu dépourvu de protéines ayant une viscosité identique à celle du liquide synovial) selon N. Gerwin et al., Advanced Drug Delivery reviews, 2006, 58, 226-242 (dosage une fois par semaine pendant 12 semaines, avec résultats cumulatifs à chaque fois). Les particules de polymère de l'invention ont été ajoutées à ce milieu à 37°C, sous agitation, puis centrifugé pour récupérer l'acide hyaluronique libéré, et doser l'acide hyaluronique libéré par la méthode de dosage de Stain-All (dosage colorimétrique du complexe Stain-All à 460 nm) (Fagnola M. et al., Contact Lens & Anterior Eye, 2009, 108-112 ; Langeslay D. J. et al., Proteoglycans : Methods and Protocols, Methods in Molecular Biology, Vol. 836, Chap. 9, pp. 131-143). Pour extraire et doser l'acide hyaluronique, 10 mg de particules de polymère selon l'invention sont mélangés pendant 30 secondes avec 2 mL de CH₂Cl₂ sous une agitation de type vortex (Vortex Genie 2, Scientific Industries, Bohemia, NY, USA), mis au repos pendant 5 min, avant l'ajout de 2 mL d'eau sous une agitation de type vortex, mis au repos pendant 30 min, puis centrifugé à une vitesse de 3500 tours/min pendant 5 min à 4°C (Centrifugeuse Jouan Thermo CR3). 150 µL de la phase aqueuse récupérée sont mélangés pendant 30 secondes avec 1,850 mL d'eau à un pH de 7,4 sous une agitation de type vortex (Vortex Genie 2, Scientific Industries, Bohemia, NY, USA). Une solution de Stain-All (1 mL) est ensuite ajoutée au milieu, et un dosage est réalisé à une absorbance de 640 nm avec un spectrophotomètre UV-visible, à l'aide d'une courbe étalon d'acide hyaluronique.

Des évaluations de tolérance *in vivo* ont également été menées sur une composition pharmaceutique préparées selon l'Exemple 1. Cette composition est injectée au sein de l'espace intra-articulaire chez le lapin, et l'effet sur la membrane synoviale et sur le liquide synovial a été étudié. Les analyses quantitatives et semi-quantitatives des effets tissulaires ont été réalisées selon la norme ISO 10993-6, sur différentes cellules circulantes du système immunitaire. Les résultats obtenus sur la membrane synoviale sont présentés au **Tableau 3,** et ceux obtenus sur le liquide synovial sont présentés au **Tableau 4.**

**Tableau 3 :**

| | **Score de l'effet de l'injection des particules de polymère selon l'invention sur la membrane synoviale** | | | | | | | |
|---|---|---|---|---|---|---|---|---|
| | 0 : Absent ; 1 : Léger ; 2 : Modéré ; 3 : Marqué ; 4 : Sévère | | | | | | | |
| Produit injecté | **Particules de polymère obtenues selon l'Exemple 1** | | **PBS** | **Sinovial** | **Particules de polymère obtenues selon l'Exemple 1** | | **PBS** | **Sinovial®** |
| Volume d'injection | 0,4 mL | | | | 0,4 mL | | | |
| Concentration du produit | 150 mg particules*/ mL | 15 mg particules*/ mL | | 8 mg d'acide hyaluroni que/mL | 150 mg de particules* / mL | 15 mg de particules */ mL | - | 8 mg d'acide hyaluroni que/mL |
| mg d'acide hyaluronique administrée *(25µg acide hyaluronique/mg particules) | 1,5 | 0,15 | - | 3,2 | 1,5 | 0,15 | - | 3,2 |
| Jour post-injections | **J3** | | | | **J7** | | | |
| Cellules polymorphonucléaires | 2 | 0,7 | 0 | 0 | 1 | 0,3 | 0 | 0 |
| Lymphocytes | 0,7 | 0 | 0 | 0 | 0,3 | 0 | 0 | 0 |
| Plasmocytes | 0 | 0 | 0 | 0 | 0 | 0 | 0 | 0 |
| Macrophages | 2 | 1,3 | 0 | 1 | 1,3 | 0,7 | 0 | 0 |
| Cellules géantes multinuclées | 1,7 | 1,3 | 0 | 0 | 1,3 | 0,3 | 0 | 0 |
| **Score global/4** | **1,28** | **0,66** | **0** | **0,2** | **0,78** | **0,26** | **0** | **0** |
| Aspect visuel de la membrane | 0,3 | 0 | 0 | 0 | 0 | 0 | 0 | 0 |
| Nécrose | 0,3 | 0 | 0 | 0 | 0 | 0 | 0 | 0 |
| Fibrose | 2 | 1,3 | 1 | 0 | 1,3 | 1 | 0 | 0 |
| Néovascularisation | 2 | 1,3 | 0 | 0 | 1,3 | 1 | 0 | 0 |
| Vésicule lipidique | 0 | 0 | 0 | 0 | 0 | 0 | 0 | 0 |
| Fibrine | 2 | 1 | 0 | 0 | 1,3 | 0,3 | 0 | 0 |
| Dégénérescence | 1,3 | 0,7 | 0 | 0 | 1 | 0,3 | 0 | 0 |
| Hyperplasie | 1,7 | 1,7 | 0 | 0 | 2 | 1 | 0 | 0 |
| Hypertrophie | 2 | 1,7 | 0 | 1 | 2 | 1,3 | 0 | 1 |
| Erosion | 2 | 1,3 | 0 | 0 | 1,7 | 0,3 | 0 | 0 |

Ces résultats montrent que la composition selon l'invention injectée est bien tolérée par la membrane synoviale de l'articulation du lapin. A la plus forte dose, le léger effet inflammatoire observé au 3^{ème} jour disparaît au 7^{ème} jour.

**Tableau 4 :**

| | **Score de l'effet de l'injection des particules de polymère selon l'invention sur le liquide synovial** | | | | | | | |
|---|---|---|---|---|---|---|---|---|
| | 0 : Absent ; 1 : Léger ; 2 : Modéré ; 3 : Marqué ; 4 : Sévère | | | | | | | |
| Produit injecté | **Particules de polymère obtenues selon l'Exemple 1** | | **PBS** | **Sinovial** | **Particules de polymère obtenues selon l'Exemple 1** | | **PBS** | **Sinovial®** |
| Volume d'injection | 0,4 mL | | | | 0,4 mL | | | |
| Concentration du produit | 150 mg particules*/ mL | 15 mg particules*/ mL | - | 8 mg d'acide hyaluroni que/mL | 150 mg de particules*/ mL | 15 mg de particules*/ mL | | 8 mg d'acide hyaluroniq ue/mL |
| ng d'acide hyaluronique administrée *(25µg acide hyaluronique/mg particules) | 1,5 | 0,15 | - | 3,2 | 1,5 | 0,15 | - | 3,2 |
| Jour post-injections | **J3** | | | | **J7** | | | |
| Cellules polymorphonucléaires | 1 | 1,3 | 0 | 0 | 0,3 | 0 | 0 | 0 |
| Lymphocytes | 0,7 | 0,7 | 0 | 1 | 0,7 | 0 | 0 | 0 |
| Plasmocytes | 0 | 0 | 0 | 0 | 0 | 0 | 0 | 0 |
| Macrophages | 1,7 | 1,3 | 0 | 1 | 0,7 | 0,5 | 0 | 1 |
| Cellules géantes multinuclées | 0 | 0 | 0 | 0 | 0 | 0 | 0 | 0 |
| **Score global/4** | **0,68** | **0,66** | **0** | **0,4** | **0,34** | **0,1** | **0** | **0,2** |
| Fibrine | 0 | 0 | 0 | 0 | 0 | 0 | 0 | 0 |
| Globules rouges | 1 | 1 | 0 | 0 | 0,3 | 0,5 | 0 | 1 |
| Cellules synoviales | 2 | 2 | 1 | 1 | 1 | 1 | 1 | 1 |

Ces résultats montrent que la composition selon l'invention injectée est bien tolérée sur le liquide synovial du lapin.

Des mesures macroscopiques de l'inflammation de l'articulation du genou du lapin ont ensuite été réalisées, là où la composition selon l'invention a été injectée. L'épaisseur de l'articulation par rapport au jour 0 **(J0)** a été mesurée à l'aide d'un pied à coulisse. Les « mesures œdèmes macroscopiques (%/**J0**) » présentées au **Tableau 5** ci-dessous représentent l'augmentation en % du volume de l'œdème par rapport à **J0.**

**Tableau 5 :**

| | **Mesure œdème macroscopique (%/J0)** | | | | | | | |
|---|---|---|---|---|---|---|---|---|
| Produit injecté | **Particules de polymère obtenues selon l'Exemple 1** | | **PBS** | **Sinovial** | **Particules de polymère obtenues selon l'Exemple** | | **PBS** | **Sinovial®** |
| Volume injection | 0,4 mL | | | | 0,4 mL | | | |
| Concentration du produit | 150 mg particules*/mL | 15 mg * particules / mL | - | 8 mg d'acide hyaluron ique/mL | 150 mg de particules*/ mL | 15 mg de particules*/m L | - | 8 mg d'acide hyaluroni que/mL |
| mg d'acide hyaluronique administrée *(25µg acide hyaluronique/mg particules) | 1,5 | 0,15 | - | 3,2 | 1,5 | 0,15 | - | 3,2 |
| Jour post-injections | **J3** | | | | **J7** | | | |
| Mesure œdème macroscopique (%/**J0**) | 1,00 | 1,00 | N.D. | 1,02 | 1,02 | 1,02 | N.D. | 1,03 |

| | | | | | | | | |
|---|---|---|---|---|---|---|---|---|
| N.D. : non déterminé | | | | | | | | |

Ces résultats montrent que la composition selon l'invention injectée est bien tolérée sur l'articulation du genou du lapin, et n'induit pas d'œdème macroscopique.

### Exemple 2 :

### Préparation des particules de polymère selon l'invention :

Un procédé similaire à celui de l'Exemple 1 est mis en œuvre pour préparer des particules de polymère selon l'invention. La seule variation qui a été faite par rapport au procédé de l'Exemple 1 est la réalisation d'une émulsion « secondaire » lors de la 2^{ème} étape à l'aide d'un agitateur Silverson à une vitesse de 2800 tours/minute, en lieu et place d'une agitation magnétique à 750 tours/minute dans l'Exemple 1.

### Analyse des particules de polymère de l'invention selon l'Exemple 2 :

Les analyses ont été réalisées sur 10 lots de particules de polymère selon l'invention. Les résultats obtenus sont les suivants :
- taille des particules déterminée par polydispersité selon la granulométrie à l'aide d'un compteur Coulter Multisizer® 3 (Beckman Coulter) : 34,98 µm ± 7,02 µm (avant irradiation) et 34,12 µm ± 7,23 µm (après irradiation),
- un taux de charge en acide hyaluronique de 16 µg d'acide hyaluronique par mg de particules de polymère lyophilisées, et
- un rendement d'association des molécules d'acide hyaluronique aux particules de polymère d'environ 20%.

Les résultats obtenus sont récapitulés dans le **Tableau 6** (taille des particules chargées en acide hyaluronique, rendement d'association des molécules d'acide hyaluronique aux particules de polymère, taux de charge des particules en acide hyaluronique).

**Tableau 6 :**

| | | Taille Moyenne (µm) | | Rendement d'association des molécules d'acide hyaluronique aux particules de polymère (%) | | Taux de charge (µg/mg) | |
|---|---|---|---|---|---|---|---|
| | | Avant irradiation | Après irradiation | Avant irradiation | Après irradiation | Avant irradiation | Après irradiation |
| **Lots** | 1 | 35,54 | 31,45 | 29 | 27 | 23,72 | 22,09 |
| | 2 | 37,62 | 32,56 | 20 | 21 | 16,36 | 17,18 |
| | 3 | 39,83 | 41,25 | 18 | 18 | 14,72 | 14,72 |
| | 4 | 34,68 | 30,63 | 24 | 26 | 19,63 | 21,27 |
| | 5 | 44,25 | 41,57 | 17 | 18 | 13,91 | 14,72 |
| | 6 | 20,19 | 25,74 | 15 | 14 | 12,27 | 11,45 |
| | 7 | 25,22 | 22,12 | 20 | 21 | 16,36 | 17,18 |
| | 8 | 32,47 | 35,89 | 18 | 17 | 14,72 | 13,91 |
| | 9 | 41,56 | 47,34 | 19 | 19 | 15,54 | 15,54 |
| | 10 | 38,47 | 32,69 | 20 | 22 | 16,36 | 18 |
| **Moyenne** | | 34,98 | 34,12 | 20,00 | 20,30 | 16,36 | 16,61 |
| **Ecart-type** | | 7,02 | 7,23 | 3,74 | 3,80 | 3,06 | 3,11 |

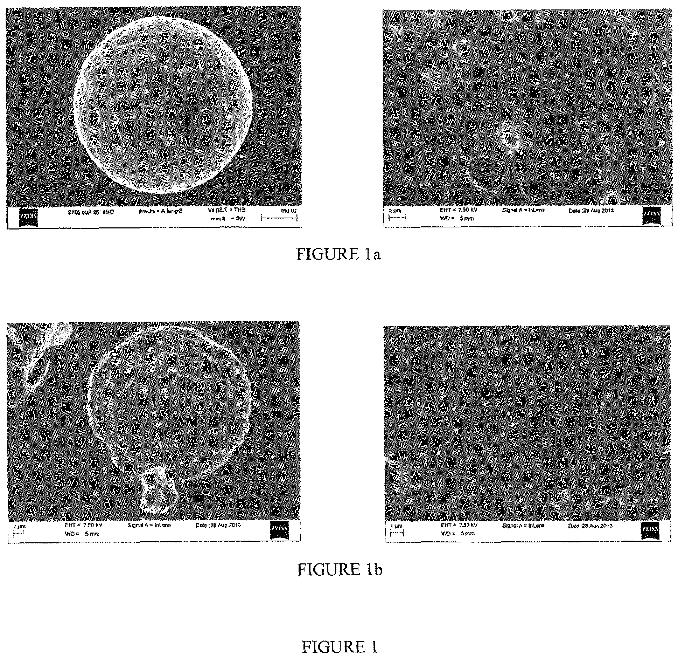

Des images de particules de polymère associées à des molécules d'acide hyaluronique selon l'invention préparées selon l'Exemple 2 sont représentées à la **Figure 1b****.**

### Résultats :

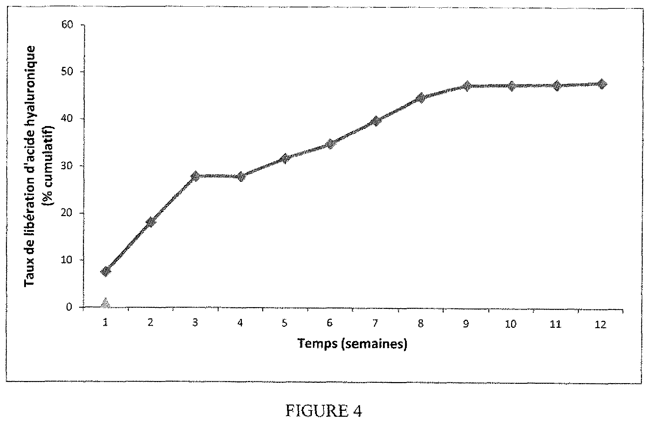

Le profil de libération des molécules d'acide hyaluronique associées à des particules de polymère préparées selon l'Exemple 2 est représenté à la **Figure 4****.**

### Exemple 3 :

### Préparation des particules de polymère selon l'invention :

Un procédé similaire à celui de l'Exemple 2 est mis en œuvre pour préparer des particules de polymère selon l'invention. La seule variation qui a été faite par rapport au procédé de l'Exemple 2 est l'ajout d'un excipient pharmaceutiquement acceptable ayant également des propriétés anti-inflammatoires (capteur de radicaux libres), à savoir 1 mL de sorbitol sous forme d'une solution de sorbitol à 10% en poids dans de l'eau ultra-pure, en lieu et place de l'eau ultra-pure utilisée dans l'Exemple 2.

### Analyse des particules de polymère de l'invention selon l'Exemple 3 :

Les analyses ont été réalisées sur 6 lots de particules de polymère selon l'invention. Les résultats obtenus sont les suivants :
- taille des particules déterminée par polydispersité selon la granulométrie à l'aide d'un compteur Coulter Multisizer® 3 (Beckman Coulter) : 31,15 µm ± 7,80 µm, et
- un taux de charge en acide hyaluronique de 13,94 µg d'acide hyaluronique par mg de particules de polymère lyophilisées.

**Tableau 7 :**

| | | Taille Moyenne (µm) | Taux de charge (µg/mg) |
|---|---|---|---|
| | | Après irradiation | Après irradiation |
| **Lots** | 1 | 32,23 | 12,17 |
| | 2 | 21,12 | 9,03 |
| | 3 | 43,55 | 15,14 |
| | 4 | 25,64 | 14,13 |
| | 5 | 29,33 | 15,32 |
| | 6 | 35,02 | 17,82 |
| **Moyenne** | | 31,15 | 13,94 |
| **Ecart-type** | | 7,80 | 3,02 |

### Exemple 4 :

### Préparation des particules de polymère selon l'invention :

Un procédé similaire à celui de l'Exemple 1 est mis en œuvre pour préparer des particules de polymère selon l'invention. La seule variation qui a été faite par rapport au procédé de l'Exemple 1 est l'ajout d'un excipient pharmaceutiquement acceptable ayant également des propriétés anti-inflammatoires (capteur de radicaux libres), à savoir 1 mL de sorbitol sous forme d'une solution de sorbitol à 10% en poids dans de l'eau ultra-pure, en lieu et place de l'eau ultra-pure utilisée dans l'Exemple 1.

### Analyse des particules de polymère de l'invention selon l'Exemple 4 :

Les analyses ont été réalisées sur 6 lots de particules de polymère selon l'invention. Les résultats obtenus sont les suivants :
- taille des particules déterminée par polydispersité selon la granulométrie à l'aide d'un compteur Coulter Multisizer® 3 (Beckman Coulter) : 71,39 µm ± 7,70 µm, et
- un taux de charge en acide hyaluronique de 23,67 µg d'acide hyaluronique par mg de particules de polymère lyophilisées.

**Tableau 8 :**

| | | Taille Moyenne (µm) | Taux de charge (µg/mg) |
|---|---|---|---|
| | | Après irradiation | Après irradiation |
| **Lots** | 1 | 65,82 | 23,61 |
| | 2 | 72,37 | 25,57 |
| | 3 | 63,92 | 22,19 |
| | 4 | 85,17 | 26,03 |
| | 5 | 67,61 | 22,73 |
| | 6 | 73,45 | 21,91 |
| **Moyenne** | | 71,39 | 23,67 |
| **Ecart-type** | | 7,70 | 1,75 |

## Revendications

1. Particules de polymère constituées d'au moins un copolymère d'acide polylactique-co-glycolique-polyéthylène glycol-acide polylactique-co-glycolique (PLGA-PEG-PLGA), ou d'un mélange de polymère d'acide polylactique-co-glycolique (PLGA) et de copolymère d'acide polylactique-co-glycolique-polyéthylène glycol-acide polylactique-co-glycolique (PLGA-PEG-PLGA) dont le ratio en poids entre le polymère d'acide polylactique-co-glycolique (PLGA) et le copolymère d'acide polylactique-co-glycolique-polyéthylène glycol-acide polylactique-co-glycolique (PLGA-PEG-PLGA) varie de 40/60 à 60/40, associées à des molécules d'acide hyaluronique ou de sels d'acide hyaluronique.

2. Particules de polymère selon la revendication 1, lesdites particules étant constituées uniquement de copolymère d'acide polylactique-co-glycolique-polyéthylène glycol-acide polylactique-co-glycolique (PLGA-PEG-PLGA).

3. Particules de polymère selon la revendication 1, lesdites particules étant constituées d'un mélange de polymère d'acide polylactique-co-glycolique (PLGA) et de copolymère d'acide polylactique-co-glycolique-polyéthylène glycol-acide polylactique-co-glycolique (PLGA-PEG-PLGA), le ratio en poids entre le polymère d'acide polylactique-co-glycolique (PLGA) et le copolymère d'acide polylactique-co-glycolique-polyéthylène glycol-acide polylactique-co-glycolique (PLGA-PEG-PLGA) étant égal à 50/50.

4. Particules de polymère selon l'une des revendications 1 à 3, dans lesquelles le taux en poids d'acide hyaluronique ou de sels d'acide hyaluronique associé aux particules de polymère varie de 1 à 50 µg.mg⁻¹, de préférence de 5 à 30 µg.mg⁻¹, et encore plus préférentiellement de 10 à 30 µg.mg⁻¹.

5. Procédé de préparation de particules de polymère telles que définies selon l'une des revendications 1 à 4 comprenant les étapes suivantes :
(i) sous agitation, mise en émulsion d'une solution aqueuse d'acide hyaluronique ou de sels d'acide hyaluronique dans une solution organique comprenant au moins un copolymère d'acide polylactique-co-glycolique-polyéthylène glycol-acide polylactique-co-glycolique (PLGA-PEG-PLGA), ou un mélange de polymère d'acide polylactique-co-glycolique (PLGA) et de copolymère d'acide polylactique-co-glycolique-polyéthylène glycol-acide polylactique-co-glycolique (PLGA-PEG-PLGA),
(ii) sous agitation, mélange de l'émulsion obtenue à l'issue de l'étape (i) avec une solution aqueuse d'alcool polyvinylique (PVA),
(iii) sous agitation, ajout d'un solvant aqueux dans l'émulsion obtenue à l'issue de l'étape (ii), ledit solvant aqueux étant de préférence de l'eau purifiée,
(iv) filtration de l'émulsion obtenue à l'issue de l'étape (iii),
(v) lyophilisation des particules de polymère obtenues à l'issu de l'étape (iv),
(vi) éventuellement, stérilisation des particules de polymère lyophilisées obtenues à l'issue de l'étape (v), de préférence aux rayons gamma.

6. Procédé selon la revendication 5, dans lequel une solution de reprise comprenant un excipient pharmaceutiquement ou cosmétiquement acceptable et/ou un autre principe actif est ajoutée aux particules de polymère obtenues à l'issu de l'étape (iv), avant d'être lyophilisées selon l'étape (v), ladite solution de reprise étant de préférence de l'eau purifiée, ledit excipient étant de préférence choisi parmi le sorbitol, le mannitol, le tréhalose, et ledit autre principe actif étant de préférence un agent anesthésique.

7. Composition comprenant des particules telles que définies selon l'une des revendications 1 à 4 en suspension dans une solution de reprise, ladite solution de reprise étant de préférence une solution d'eau purifiée, une solution aqueuse d'acide hyaluronique (réticulé ou non réticulé) ou de sels d'acide hyaluronique, une solution aqueuse d'un autre sel, et plus préférentiellement une solution aqueuse d'acide hyaluronique ou de sels d'acide hyaluronique dont la concentration en poids d'acide hyaluronique ou de sels d'acide hyaluronique varie de 0,5 à 10%.

8. Composition selon la revendication 7, ladite composition comprenant également un excipient pharmaceutiquement ou cosmétiquement acceptable, de préférence choisi parmi le sorbitol, le mannitol, le tréhalose.

9. Composition selon la revendication 7 ou la revendication 8, ladite composition comprenant également un autre principe actif, de préférence un agent anesthésique.

10. Procédé de préparation d'une composition telle que définie selon l'une des revendications 7 à 9, comprenant les étapes (i) à (vi) telles que définies précédemment aux revendications 5 et 6, et une étape (vii) de mise en suspension des particules de polymère lyophilisées obtenues à l'issue de l'étape (v) ou (vi) dans une solution de reprise telle que définie à la revendication 7, ladite solution de reprise pouvant comprendre un excipient pharmaceutiquement ou cosmétiquement acceptable tel que défini à la revendication 8 et/ou un autre principe actif tel que défini à la revendication 9.

11. Composition selon l'une des revendications 7 à 9, pour son utilisation à titre de médicament.

12. Composition pour son utilisation selon la revendication 11, pour la prévention et/ou le traitement des maladies musculo-squelettiques telles que l'arthrose, et plus particulièrement la gonarthrose ou l'arthrose sans épanchement ; des maladies et des états traumatiques de la peau ; des affections bucco-dentaires ; de la sécheresse de la muqueuse vaginale et des infections urinaires ou cystites ; de la sécheresse de la membrane oculaire et des infections oculaires ; et de l'obésité.

13. Utilisation d'une composition selon l'une des revendications 7 à 9, pour lutter contre le vieillissement de la peau et/ou pour réparer le tissu dermique (mésothérapie).

14. Kit comprenant :
(a) un premier contenant comprenant des particules de polymère telles que définies selon l'une des revendications 1 à 4, et
(b) un deuxième contenant comprenant une solution de reprise telle que définie à la revendication 7, ladite solution de reprise pouvant comprendre un excipient pharmaceutiquement ou cosmétiquement acceptable tel que défini à la revendication 8 et/ou un autre principe actif tel que défini à la revendication 9.

15. Seringue prête à l'emploi comprenant une composition telle que définie selon l'une des revendications 7 à 9.

## Patentansprüche

1. Polymerpartikel, bestehend aus zumindest einem Copolymer Polymilchsäure-co-Glycolid-Polyethylenglykol-Polymilchsäure-co-Glycolid (PLGA-PEG-PLGA) oder aus einer Mischung des Polymers Polymilchsäure-co-Glycolid (PLGA) und des Copolymers Polymilchsäure-co-Glycolid-Polyethylenglykol-Polymilchsäure-co-Glycolid (PLGA-PEG-PLGA), deren Gewichtsverhältnis zwischen dem Polymer Polymilchsäure-co-Glycolid (PLGA) und dem Copolymer Polymilchsäure-co-Glycolid-Polyethylenglykol-Polymilchsäure-co-Glycolid (PLGA-PEG-PLGA) von 40/60 bis 60/40 variiert, wobei die Partikel mit Hyaluronsäuremolekülen oder Salzen der Hyaluronsäure assoziiert sind.

2. Polymerpartikel nach Anspruch 1, wobei die Partikel ausschließlich aus dem Copolymer Polymilchsäure-co-Glycolid-Polyethylenglykol-Polymilchsäure-co-Glycolid (PLGA-PEG-PLGA) bestehen.

3. Polymerpartikel nach Anspruch 1, wobei die Partikel aus einer Mischung des Polymers Polymilchsäure-co-Glycolid (PLGA) und des Copolymers Polymilchsäure-co-Glycolid-Polyethylenglykol-Polymilchsäure-co-Glycolid (PLGA-PEG-PLGA) bestehen, wobei das Gewichtsverhältnis zwischen dem Polymer Polymilchsäure-co-Glycolid (PLGA) und dem Copolymer Polymilchsäure-co-Glycolid-Polyethylenglykol-Polymilchsäure-co-Glycolid (PLGA-PEG-PLGA) gleich 50/50 ist.

4. Polymerpartikel nach einem der Ansprüche 1 bis 3, wobei der Gewichtsanteil der Hyaluronsäure oder der Salze der Hyaluronsäure, die mit den Polymerpartikeln assoziiert ist/sind, von 1 bis 50 µg.mg⁻¹, vorzugsweise von 5 bis 30 µg.mg⁻¹, und noch bevorzugter von 10 bis 30 µg.mg⁻¹ variiert.

5. Verfahren zur Herstellung von Polymerpartikeln, wie sie nach einem der Ansprüche 1 bis 4 definiert sind, umfassend die folgenden Schritte:
(i) Herstellen einer Emulsion aus einer wässrigen Lösung von Hyaluronsäure oder von Salzen der Hyaluronsäure in einer organischen Lösung, die zumindest ein Copolymer Polymilchsäure-co-Glycolid-Polyethylenglykol-Polymilchsäure-co-Glycolid (PLGA-PEG-PLGA) oder eine Mischung des Polymers Polymilchsäure-co-Glycolid (PLGA) und des Copolymers Polymilchsäure-co-Glycolid-Polyethylenglykol-Polymilchsäure-co-Glycolid (PLGA-PEG-PLGA) umfasst, unter Rühren,
(ii) Mischen der am Ende von Schritt (i) erhaltenen Emulsion mit einer wässrigen Lösung von Polyvinylalkohol (PVA) unter Rühren,
(iii) Zugeben eines wässrigen Lösungsmittels zu der am Ende von Schritt (ii) erhaltenen Emulsion unter Rühren, wobei das wässrige Lösungsmittel vorzugsweise gereinigtes Wasser ist,
(iv) Filtrieren der am Ende von Schritt (iii) erhaltenen Emulsion,
(v) Lyophilisieren der am Ende von Schritt (iv) erhaltenen Polymerpartikel,
(vi) optional Sterilisieren der am Ende von Schritt (v) erhaltenen lyophilisierten Polymerpartikel, vorzugsweise mittels Gammastrahlen.

6. Verfahren nach Anspruch 5, wobei eine Wiederaufbereitungslösung, umfassend einen pharmazeutisch oder kosmetisch akzeptablen Exzipienten und/oder einen anderen Wirkstoff, zu den am Ende von Schritt (iv) erhaltenen Polymerpartikeln zugegeben wird, bevor diese gemäß Schritt (v) lyophilisiert werden, wobei die Wiederaufbereitungslösung vorzugsweise gereinigtes Wasser ist, wobei der Exzipient vorzugsweise aus Sorbitol, Mannitol und Trehalose ausgewählt wird, und der andere Wirkstoff vorzugsweise ein Anästhetikum ist.

7. Zusammensetzung, umfassend Partikel, wie sie nach einem der Ansprüche 1 bis 4 definiert sind, in Suspension in einer Wiederaufbereitungslösung, wobei die Wiederaufbereitungslösung vorzugsweise eine Lösung aus gereinigtem Wasser, einer wässrigen Lösung von Hyaluronsäure (vernetzt oder nicht vernetzt) oder von Salzen der Hyaluronsäure und einer wässrigen Lösung aus einem anderen Salz ist, und noch bevorzugter einer wässrigen Lösung von Hyaluronsäure oder von Salzen der Hyaluronsäure, deren Konzentration an Hyaluronsäure oder an Salzen der Hyaluronsäure nach Gewicht von 0,5 bis 10 % variiert.

8. Zusammensetzung nach Anspruch 7, wobei die Zusammensetzung auch einen pharmazeutisch oder kosmetisch akzeptablen Exzipienten umfasst, der vorzugsweise aus Sorbitol, Mannitol und Trehalose ausgewählt ist.

9. Zusammensetzung nach Anspruch 7 oder Anspruch 8, wobei die Zusammensetzung auch einen weiteren Wirkstoff, vorzugsweise ein Anästhetikum umfasst.

10. Verfahren zur Herstellung einer Zusammensetzung, wie sie nach einem der Ansprüche 7 bis 9 definiert ist, umfassend die Schritte (i) bis (vi), wie sie vorstehend in den Ansprüchen 5 und 6 definiert sind, sowie einen Schritt (vii) des Herstellens einer Suspension der am Ende von Schritt (v) oder (vi) erhaltenen lyophilisierten Polymerpartikel in einer Wiederaufbereitungslösung, wie sie in Anspruch 7 definiert ist, wobei die Wiederaufbereitungslösung einen pharmazeutisch oder kosmetisch akzeptablen Exzipienten, wie er in Anspruch 8 definiert ist, und/oder einen anderen Wirkstoff, wie er in Anspruch 9 definiert ist, umfassen kann.

11. Zusammensetzung nach einem der Ansprüche 7 bis 9 zur Verwendung als Medikament.

12. Zusammensetzung zur Verwendung nach Anspruch 11 für die Vorbeugung und/oder die Behandlung von Krankheiten des Bewegungsapparats, wie Arthrose, und insbesondere Gonarthrose oder Arthrose ohne Schwellung/Erguss, traumatischen Krankheiten und Zuständen der Haut, Erkrankungen im Mund- und Zahnbereich, Trockenheit der Vaginalschleimhaut und Infektionen des Urogenitaltrakts oder Cystitis, Trockenheit der Augenmembran und Infektionen am Auge, sowie der Adipositas.

13. Verwendung einer Zusammensetzung nach einem der Ansprüche 7 bis 9 zur Bekämpfung der Hautalterung und/oder zur Reparatur des Hautgewebes (Mesotherapie).

14. Kit, umfassend:
(a) einen ersten Behälter, umfassend Polymerpartikel, wie sie nach einem der Ansprüche 1 bis 4 definiert sind, und
(b) einen zweiten Behälter, umfassend eine Wiederaufbereitungslösung, wie sie in Anspruch 7 definiert ist, wobei die Wiederaufbereitungslösung einen pharmazeutisch oder kosmetisch akzeptablen Exzipienten, wie er in Anspruch 8 definiert ist, und/oder einen anderen Wirkstoff, wie er in Anspruch 9 definiert ist, umfassen kann.

15. Gebrauchsfertige Spritze, umfassend eine Zusammensetzung, wie sie nach einem der Ansprüche 7 bis 9 definiert ist.

## Claims

1. Polymer particles composed of at least a poly(lactic-co-glycolic acid)-polyethylene glycol-poly(lactic-co-glycolic acid) (PLGA-PEG-PLGA) copolymer, or a mixture of poly(lactic-co-glycolic acid) (PLGA) polymer and of poly(lactic-co-glycolic acid)-polyethylene glycol-poly(lactic-co-glycolic acid) (PLGA-PEG-PLGA) copolymer, in which the ratio by weight of the poly(lactic-co-glycolic acid) (PLGA) polymer to the poly(lactic-co-glycolic acid)-polyethylene glycol-poly(lactic-co-glycolic acid) (PLGA-PEG-PLGA) copolymer varies from 40/60 to 60/40, combined with hyaluronic acid molecules or hyaluronic acid salts.

2. The polymer particles according to claim 1, said particles consisting solely of poly(lactic-co-glycolic acid)-polyethylene glycol-poly(lactic-co-glycolic acid) (PLGA-PEG-PLGA) copolymer.

3. The polymer particles according to claim 1, said particles consisting of a mixture of poly(lactic-co-glycolic acid) (PLGA) polymer and of poly(lactic-co-glycolic acid)-polyethylene glycol-poly(lactic-co-glycolic acid) (PLGA-PEG-PLGA) copolymer, the ratio by weight of the poly(lactic-co-glycolic acid) (PLGA) polymer to the poly(lactic-co-glycolic acid)-polyethylene glycol-poly(lactic-co-glycolic acid) (PLGA-PEG-PLGA) copolymer being equal to 50/50.

4. The polymer particles according to any one of claims 1 to 3, in which the content by weight of hyaluronic acid or of hyaluronic acid salts combined with the polymer particles varies from 1 to 50 µg.mg⁻¹, preferably from 5 to 30 µg.mg⁻¹ and more preferably from 10 to 30 µg.mg⁻¹.

5. A process for the preparation of polymer particles as defined according to any one of claims 1 to 4, comprising the following stages:
(i) with stirring, emulsifying an aqueous solution of hyaluronic acid or of hyaluronic acid salts in an organic solution comprising at least a poly(lactic-co-glycolic acid)-polyethylene glycol-poly(lactic-co-glycolic acid) (PLGA-PEG-PLGA) copolymer, or a mixture of poly(lactic-co-glycolic acid) (PLGA) polymer and of poly(lactic-co-glycolic acid)-polyethylene glycol-poly(lactic-co-glycolic acid) (PLGA-PEG-PLGA) copolymer,
(ii) with stirring, mixing the emulsion obtained from stage (i) with an aqueous solution of polyvinyl alcohol (PVA),
(iii) with stirring, adding an aqueous solvent to the emulsion obtained from stage (ii), said aqueous solvent preferably being purified water,
(iv) filtering the emulsion obtained from stage (iii),
(v) freeze-drying the polymer particles obtained from stage (iv),
(vi) optionally sterilizing the freeze-dried polymer particles obtained from stage (v), preferably with gamma rays.

6. The process according to claim 5, in which an uptake solution comprising a pharmaceutically or cosmetically acceptable excipient and/or another active principle is added to the polymer particles obtained from stage (iv), before being freeze-dried according to stage (v), said uptake solution preferably being purified water, said excipient preferably being chosen from sorbitol, mannitol, trehalose, and said other active principle preferably being an anesthetic agent.

7. A composition comprising particles as defined according to any one of claims 1 to 4 in suspension in an uptake solution, said uptake solution preferably being a solution of purified water, an aqueous solution of hyaluronic acid (crosslinked or not crosslinked) or of hyaluronic acid salts, an aqueous solution of another salt, and more preferably an aqueous solution of hyaluronic acid or of hyaluronic acid salts in which the concentration by weight of hyaluronic acid or of hyaluronic acid salts varies from 0.5 to 10%.

8. The composition according to claim 7, said composition also comprising a pharmaceutically or cosmetically acceptable excipient, preferably chosen from sorbitol, mannitol, trehalose.

9. The composition according to any one of claims 7 or 8, said composition also comprising another active principle, preferably an anesthetic agent.

10. A process for the preparation of a composition as defined according to any one of claims 7 to 9, comprising stages (i) to (vi) as defined above in claims 5 and 6 and a stage (vii) of suspending the freeze-dried polymer particles obtained from stage (v) or (vi) in an uptake solution as defined in claim 7, it being possible for said uptake solution to comprise a pharmaceutically or cosmetically acceptable excipient as defined in claim 8 and/or another active principle as defined in claim 9.

11. The composition according to any one of claims 7 to 9, for use thereof as medicament.

12. The composition for use according to claim 11, in the prevention and/or treatment of musculoskeletal disorders such as osteoarthritis, and more particularly osteoarthritis of the knee or effusion-free osteoarthritis ; diseases and traumatic states of the skin; oral conditions; dryness of the vaginal mucous membrane and urinary infections or cystitis; dryness of the ocular membrane and ocular infections; and obesity.

13. The use of the composition according to any one of claims 7 to 9, for combating aging of the skin and/or for repairing dermal tissue (mesotherapy).

14. A kit comprising:
(a) a first container comprising polymer particles as defined according to any one of claims 1 to 4, and
(b) a second container comprising an uptake solution as defined according to claim 7, it being possible for said uptake solution to comprise a pharmaceutically or cosmetically acceptable excipient as defined according to claim 8 and/or another active principle as defined according to claim 9.

15. A ready-for-use syringe, comprising a composition as defined according to any one of claims 7 to 9.
